# EUROPEAN PATENT APPLICATION

(11) **EP 0 943 341 A1**
(43) Date of publication of application: **22.09.1999**
(21) Application number: 99500020.5
(22) Date of filing: 09.02.1999
(51) Int. Cl.: A61K 47/48, A61K 31/70, A61K 9/50, A61K 9/16

(54) **Procedure to prepare granulated compositions that contain erythromycin macrolides and procedure to prepare pharmaceutical compositions that contain said macrolides**

(30) Priority: 18.02.1998 AR 9810711
(71) Applicant: Gold, Oscar, 1004 Buenos Aires (AR)
(72) Inventor: Gold, Oscar, 1004 Buenos Aires (AR)
(74) Representative: Urizar Anasagasti, José Antonio

(57) **Abstract**

Procedure to prepare granulated compositions that contain erythromycin macrolides, useful to prepare pharmaceutical formulations that can be administered orally, of acceptable organoleptic characteristics -flavor and aroma-, characterized because it includes:
- mixing a protonized resin in particles with an erythromycin macrolide in a solvent means for said compound, during a lapse enough in order to neutralize and to fix, through acid/base exchange, the macrolide, in the granules of the resin;
- to crush the granules of resin treated this way and if it is necessary, with previous elimination of the solvent;
- to classify and to select the particles of a predetermined grain measure and
- to coat the classified particles of substantially neuter aroma and flavor, with a biodegradable coating of programmed liberation of the macrolide from the particles of resin.

## Description

The present invention is related with a procedure to prepare compositions that contain erythromycin compounds under the form of particles provided of a biodegradable coating, able to hide the organoleptic characteristics of this class of compounds and to facilitate the ingestion of formulations that contain them as active agent. This procedure includes to make react the erythromycin, or derivatives of the same with a reagent of acid character, pharmaceutically acceptable and to form a compound insoluble or not very soluble in an acuous means, fissionable under the conditions of the internal means where v.gr. should be liberated said active agent being dispensed.

This way, it is possible to administer oral compositions formulated with erythromycin, of reasonably acceptable flavor and aroma and to exclude the common rejection of this class of medications.

### Antecedents of the invention

The erythromycin is an antibiotic of basic character (pKa: 8,8) soluble in water, of wide spectrum, included inside the group denominated macrolides, of extended application in human and veterinary medicine, of which derivatives have been prepared also of wide application like the semisynthetical macrolides that correspond to the esters of the erythromycin such as glycoheptonic acid, propionic acid and fatty acids: estearic acid for example and others derivatives formed by certain substitution reactions in the erythromycin molecule, as the chlaritromycin (Chlarithromycin), Roxithromycin (Roxithromycin), Azithromycin (Azithromycin), Dirithromycin (Dirithromycin).

In function of the bitter flavor and the scent of this class of compound, the frequent rejection of the medications that are administered orally (syrups, emulsions, pills or capsules) is manifested especially with pediatric medicines, rejections, many times manifested in compulsive form: nausea and vomits.

It has been found and verified now, that it is possible to prepare and to have compositions that can be administered **per os**, of acceptable organoleptic characteristics -v.gr. aroma, flavor and palatable, formulated with erythromycin, respectively with semi-synthetical macrolidic derivatives of the same, of equivalent antibacterial spectrum, in which the presence of the active agent organoleptically masked is, so much in the container as before or during the ingestion, because in the compositions formulated and prepared this way, the active agent, specifically, erythromycin and its mentioned derivatives - is distributed and immobilized in a plurality of granular particles, recovered where they are liberated in response to the physiologic conditions of the internal means.

In the text that follows reference will be made to the active principles used in the present invention, specifically, erythromycin and macrolides derivative of the same under the generic denomination of "erythromycinic macrolides" or simply "erythromycin macrolides" (that besides the Erythromycin, Roxitromycin, Azitromycin includes, esters such as estearate lactobionate erythromycin gluconeptomate, etc.

It is therefore an object of the invention: a procedure to prepare granular compositions that contain erythromycin macrolides, useful as active agent to prepare pharmaceutical compositions that may be administered orally, of organoleptic characteristics: acceptable flavor, aroma and palatability, characterized because it includes:
- to mix in a solvent means and to put in relationship of acid/base exchange, an erythromycin macrolide with protonized granulated resin, during a lapse enough to fix said macrolide in the granules of the resin;
- to classify and to select the particles in which the measuring of the grain responds to a certain pattern;
- to recover the selected granulated particles with a biodegradable coating of programmed liberation of the macrolide fixed in said particles and
- to recover the coated granular particles of programmed liberation of the macrolide, of substantially neuter aroma and flavor.

It is another object of this invention, a procedure where the obtained coated granulated particles are combine with a pharmaceutically acceptable vehicle, solid or liquid, to form syrups, emulsions or orally administered dosage units, exempt of the aroma and flavor characteristic of the erythromycin macrolides, of programmed liberation in the internal means (for example: in the gastric means, or duodenal means).

It is another object of the present invention the preparation of medicinal formulations that include macrolides of erythromycin of masked flavor and aroma; this procedure includes to disperse this product in a flavored liquid means and perfumed as it is habitual, to form syrups or other drinkable or encapsulated formulations and the preparation of medical formulations consistent of: tablets, pills, effervescent powdered products that include the macrolides of erythromycin of masked flavor and aroma with the conventional excipients and loads.

### Detailed description of the invention

In the present invention an extremely suitable and simple procedure is proposed, in order solve the problems that the conventional medicinal formulations have, for the therapies based on the oral administration of erythromycin and analogues of the same. In these cases, rejection is usually frequent, especially with treatments of certain extension, in response to the aroma, flavor and the palatability of this type of medications, administered usually under the form os syrups and solid formulations.

According to the present invention, now it is possible to have medications formulated with erythromycin macrolides, practically odorless and insipid as much as it refers to the participation of said macrolides in the organoleptic properties of the medication, since the molecules of the used macrolide are caught and immobilized in the reticular structure of the granular particles of the polymer or resin used in the first stage of the procedure-object of this invention. The erythromycin macrolides have an amino substituted group (-N(CH₃)₂ in the position 3 of one of the two pyranoside groups and this group cuaternizable labile participates in acid/base reactions modifying the reactivity and solubility of these macrolides. This has induced and driven to the applicant to take advantage of that property of the mentioned macrolides to catch and to immobilize the molecules of these compounds, beginning with the erythromycin, in an insoluble substratum able to make available to the macrolides molecules, the necessary protons for the cuaternization of the group -N(CH₃)₂ with the transitory immobilization of the macrolides, but reversible when the conditions of the external means (physiologic means v.gr.) are opposed to the quaternary condition of the group -N(CH₃)₂.

In the stage we denominate "neutralization" that is to say of coupling or combination of the macrolides with the polymeric substrate, the reaction is made in an acuous means or with polar organic solvents, indistinctly, for example ethanol, isopropanol, chlorated solvents, acetone.

The material preferred as support or substratum of the macrolides should gather the following conditions:
- it should be pharmaceutically acceptable
- to reduce the solubility of the elected macrolide in an acuous means.
- it should allow the exit of the caught macrolide, once the coating (enteric or gastric) is removed, where said active agent should be liberated.

The following step, the macrolides anchored in main matrix or mentioned support, from now on denominated acid/base complex is the drying of the same. In this aspect, the application of the conventional techniques (vacuum drying, by means of aspersion or in fluid bed) have not restriction.

In what has relationship with following stages of the process the following must be noted:
for the coating of the micro particles of the complex, dispersions of polymers in different solvents are used. The polymers can be of the type of the Eudragit (r), of cellulose ethers or esters, porydone, etc. The solvents in which these polymers are dissolved are chosen according to the polymer to be used. These solutions can also contain variable quantities of plasticizers of the type of the phtalates, esters citrates, triglycerids, etc. It is also possible to incorporate lubricant of the type of silicon dioxide, talc, magnesium estearate, etc.

According to other characteristics of the invention, the following proportions are preferable:

The proportion of active principle with regard to the agent making the complex can be from 10 to 90%.

The final concentration of active principle in the micro coated particles can be from 5 to 80%.

The final grain measure of the coated particles is variable according to its destination.
- If it is for pediatric preparations, a grain of <600 microns will be required.
- If it is for a preparation for adults (capsules, etc.), the grain may reach approximately 1mm.

The used solvents can be organic or acuous.

The type of applied coating defines the type of behavior of the particles before the breakup test type USP 23 page 1791 and certainly in the physiologic means where it is liberated.

In general the coating will allow the obtaining of the masking of the flavor.

If the selected coating is of the type of Eudragit E-100(r), this will allow a quick breakup in the acid means in the stomach.

If the selected coating is of the type enteric, this it will allow a quick breakup in the intestinal means. Coatings of this type is Eudragit L, S(r), or the cellulosic derivatives such as Hydroxypropylmethyl Cellulose Phtalate, Cellulose Acetophtalate, etc.

In the following examples included as an illustrative example the best way to carry out the invention is revealed.

### Example 1

| | |
|---|---|
| Chlaritromycin | 150 grams |
| Carbopol 934 | 150 grams |

The Chlaritromycin was dissolved in enough quantity of Acetone, and that solution was poured on the Carbopol. The resultant humid mass was dried through heating at 55°C. The obtained product, with a theoretical title of 50%, tumed out to have a diminished flavor with regard to the pure drug.

### Example 2

| | |
|---|---|
| Chlaritromycin | 150 grams |
| Carbopol 934 | 150 grams |

Both components were mixed and afterwards they were carefully kneaded with enough quantity of purified water. Evolution of heat was observed: the mixture elevated its temperature. Once dried, the resulting product presented characteristics similar to those of exampe 1.

### Example 3

| | |
|---|---|
| Chlaritromycin | 500 grams |
| Carbopol 934 | 300 grams |

Both components were mixed carefully and later on they were kneaded with enough quantity of a misture of ethanol (75%) and water (25%). The behavior was similar to that of example 2.

### Example 4

The complex obtained in example 3 was carefully milled to powder in a hammer mill. Particles included among 180 to 450 microns were selected. The particles outside of that range were processed again with solution of PVP in Isopropanol to 10%, kneading and granulating until obtaining enough quantity of particles in the wanted range of grain measure. That mass of particles was placed in a team of fluid bed in pilot scale, and proceeded to apply a coating with Hydroxypropylmethylcellulose Phtalate dissolved in a proportion of 10% in a solvent constituted by water (6%), acetone (47%) and ethanol (47%) and with the addition of an appropiate plasticizer, in this case castor oil. When it was considered that the coating was enough, a suspension of said coated particles was formulated, like it is described in the example 5.

### Example 5

### Observations:

Before proceeding to the final formulation it corresponds to verify with a test at a small scale that the pH of the reconstituted suspension is around 4.3. Given the case adjust the pH with citric acid.

### Favorite formulation:

| (Formula for 5ml. of reconstituted suspension) | |
|---|---|
| anh. citric acid | 14.0mg |
| potassium sorbate | 20.0mg |
| sodium chloride | 12.5mg |
| Sugar | 3000.0mg |
| zanthan rubber | 7.5mg |
| Insoluble saccharin | 8.0mg |
| Chlaritromycin MIC csp | 125.0mag |
| Flavoring: see observ. | (*) |

| | |
|---|---|
| (*) Observ.: Flavoring have been used such as Vanilla IFF PIR-3178 (2,5 mg) plus Raspberry IFF PIR-2179 (2,5 mg). | |

This preparation, once the suspension for pediatric use is reconstituted, maintained the masking of the flavor of the antibiotic still for fourteen days after being prepared. To demonstrate quantitative and objectively that the sum of both processes: formation of a not very soluble acid/base complex and later the coating of said complex provided a perfect masking of the flavor, we proceeded like it is described in the example 6.

### Example 6

With a small tasting panel it was demonstrated that a formulation like the one described in the example 5 can disguise the flavor coming from a concentration correctly of approximately 0.4 mg of Chlaritromycin per ml of syrup.

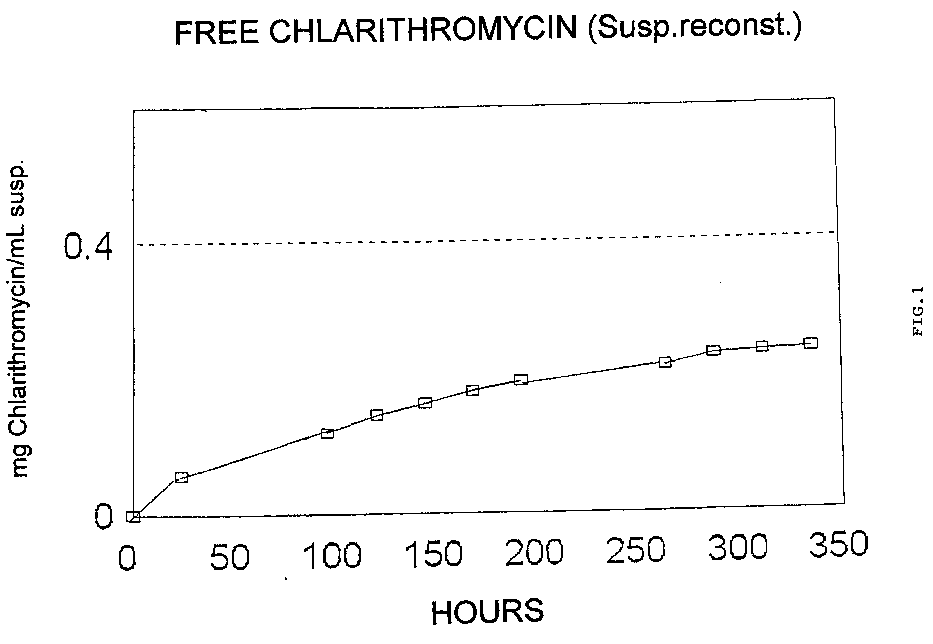

Consequently a suspension was prepared in accordance with this formulation and filtered samples of the same were removed periodically. The quantity of dissolved Chlaritromycin of these samples was determined during a lapse of 14 days. The results are illustrated in figure 1.

One can see clearly that after 14 days the liberation of Chlaritromycin from the coated micro particles is sufficiently low so as to assure an excellent masking of the flavor.

## Claims

1. Procedure to prepare granulated compositions that contain erythromycin macrolides, useful to prepare pharmaceutical formulations that can be administered orally, of acceptable organoleptic characteristics -flavor and aroma-, characterized because it includes:
- mixing a protonized resin in particles with an erythromycin macrolide in a solvent means for said compound, during a lapse enough in order to neutralize and to fix, through acid/base exchange, the macrolide, in the granules of the resin;
- to crush the granules of resin treated this way and if it is necessary, with previous elimination of the solvent;
- to classify and to select the particles of a predetermined grain measure and
- to coat the classified particles of substantially neuter aroma and flavor, with a biodegradable coating of programmed liberation of the macrolide from the particles of resin.

2. Procedure in accordance with claim 1, wherein this macrolide is an erythromycin macrolide, chlaritromycin, azitromycin, roxitromycin and erythromycin esters.

3. Procedure in accordance with claim 1, wherein this macrolide, is the glucoheptogluconate or erythromycin lactobionate.

4. Procedure in accordance with the precedent claims wherein this resin is Carbopol (a carboxyvinyl polymer) or resins of methacrylic acid).

5. Procedure in accordance with claim 1, wherein this coating is a povidone polymeric coating, cellulose esters or ethers or methyl poly(met)acrylates, obtained as from solutions or dispersions of the same that include plasticizers such as castor oil and lubricant as talc magnesium estearate.

6. Procedure in accordance with claim 1 and 5, wherein the coating is formed in fluid bed or humid means.

7. Procedure in accordance with claims 1 at 4, wherein coated particles of grain of around 600 microns are obtained for pediatric formulations or up to one millimeter in formulations for adults.

8. Procedure in accordance with claim 1, wherein this coating is an enteric coating, of breakup in the gastric means and the content of erythromycin macrolide in the recovered particles is from 5 to 80% ponderal.

9. Procedure in accordance with claim 5, wherein the coating is a coating of hydroxypropylcellulose Phtalate, cellulose or polymethylmetacrylate acetophtalate.

10. Procedure to prepare pharmaceutical compositions that contain erythromycin macrolides, of acceptable organoleptic characteristics, wherein it includes to combine predetermined quantities of the granulated composition obtained with the procedure from claims 1 to 9, with a means of dispersion pharmaceutically acceptable that includes dispersant preservatives, stabilizers and flavorants.

11. Procedure to prepare pharmaceutical compositions that contain erythromycin macrolides, of acceptable organoleptic characteristic, wherein it combines the product in particles obtained pharmaceutically in claim 1 with an acceptable vehicle and forming dosage units, consistent or encapsulated.
